Europäisches Patentamt

(19) European Patent Office

Office européen des brevets

(11) Publication number: **0 034 671**
**A1**

## (12) EUROPEAN PATENT APPLICATION

(21) Application number: 80300459.7

(22) Date of filing: 18.02.80

(51) Int. Cl.³: **A 61 M 5/14**
**A 61 M 1/03, G 05 D 7/01**
**F 16 K 7/07**

(43) Date of publication of application:
02.09.81 Bulletin 81/35

(84) Designated Contracting States:
AT BE CH DE FR IT LU NL SE

(71) Applicant: **UNIVERSITY OF EXETER**
**Northcote House The Queen's Drive**
**Exeter Devon EX4 4QJ(GB)**

(72) Inventor: **Griffiths, Derek John**
**Lijsterbes 34**
**Krimpen aan de IJssel(NL)**

(74) Representative: **Armitage, Ian Michael et al,**
**MEWBURN ELLIS & CO. 70/72 Chancery Lane**
**London WC2A 1AD(GB)**

(54) Method and apparatus, especially an intravenous infusion set, for maintaining a desired rate of fluid flow.

(57) Fluid flow rate is controlled by passing it through a collapsible conduit (22) and arranging that the pressure head driving the fluid through the fluid path downstream of the collapsible conduit is sufficiently smaller than the pressure head driving the fluid through the collapsible conduit that the conduit is maintained in a partially collapsed condition. This decouples the upstream and downstream parts of the path and prevents downstream pressure variations being propagated to the upstream part. Variations in the upstream pressure head, e.g. in a supply reservoir (10), can be compensated by transmitting those pressure variations also to an enclosure (24) around the collapsible conduit, thereby keeping the transmural pressure difference constant. An adjustable height chamber (30), partially filled with liquid, in the pressure compensation transmission path enables the flow rate to be varied. The invention is particularly applicable to intravenous infusion sets for controlling the infusion rate.

./...

Croydon Printing Company Ltd.

*Fig. 1*

-1-

## METHOD AND APPARATUS, ESPECIALLY AN INTRAVENOUS INFUSION SET, FOR MAINTAINING A DESIRED RATE OF FLUID FLOW

This invention relates to methods and apparatus for controlling fluid flow rate. It is especially applicable to controlling the flow rate of liquid in intravenous infusion sets.

Conventional intravenous infusion sets, incorporating a drip chamber, have their flow rate set at least in part by the height of the reservoir container above the patient. However the height of liquid in the reservoir will decrease with time, thereby tending to decrease the flow rate, and also changes in the venous pressure of the patient are transmitted back through the supply to alter the flow rate.

The present invention provides, for general application, a better control for fluid flow rate, which is normally unaffected by downstream back-pressures and which, in a preferred form of intravenous infusion set, can also be substantially independent of the liquid level in the reservoir. The independence from downstream pressure variations is achieved by decoupling upstream and downstream portions of the fluid flow path by passing the fluid through a partially collapsed collapsible conduit comprising a tube defined at least in part by flexible material and which can readily vary in internal cross-section according to small variations in the transmural pressure difference. More particularly, in its practical application the collapsible conduit can operate with an internal cross-section which is smaller than any cross-section in the downstream fluid flow path, so that it is capable of collapsing down to a relatively very small, preferably zero, internal cross-section.

If the fluid pressure head $P_1$ supplied to the partially collapsed collapsible conduit is sufficiently greater than the pressure head $P_2$ necessary to drive the fluid at the desired flow rate through the flow path downstream of the collapsible conduit, then the speed of the fluid just downstream of the region of maximum constriction in the collapsible conduit can exceed the speed of the pressure waves (Young waves) along the flow path. Thus pressure variations in the downstream path cannot be transmitted back through this region of "supersonic" flow speed. As the downstream fluid pressure rises the length of this supersonic region decreases, and eventually will disappear so that the two parts of the flow path are no longer decoupled and the control provided by the present invention no longer exists.

With the fluid flow control established as above, the flow rate for a given supply pressure head will depend on the condition of the collapsible conduit. Thus the flow rate might be expected to reduce as the level of liquid in a supply reservoir decreases. In a preferred arrangement, however, this is substantially avoided by transmitting the changes in pressure head to the outside of the collapsible conduit, thus tending to keep the transmural pressure difference constant.

Accordingly one aspect of the present invention provides a method of maintaining a desired rate of fluid flow, which comprises passing the fluid along a path which includes a collapsible conduit which can vary in cross-section according to variations in the transmural pressure difference, and arranging that the pressure head driving the fluid through the fluid path downstream of the collapsible conduit is sufficiently smaller than the pressure head driving the fluid through the collapsible conduit, that the collapsible conduit is partially collapsed and mean fluid pressure variations in the downstream path are not propagated upstream of the collapsible conduit.

Another aspect of the present invention provides an intravenous infusion set adapted for maintaining a desired rate of liquid flow by the foregoing method, the set comprising a first tube for connection to a giving bag or bottle, a second tube for connection to a hypodermic needle, and a drip chamber and a said collapsible conduit located in the liquid path between the two tubes.

The collapsible conduit in one form may comprise a flexible sleeve surrounding a rigid insert, the internal circumference of the sleeve in cross-section being substantially the same as the external circumference of the cross-section of the insert which it surrounds, the insert having an inlet leading to an internal passage for the fluid and a lateral outlet therefrom intermediate the ends of the insert and covered by the sleeve, a longitudinal surface portion of the insert from the region of said outlet being concave in cross-section to provide a channel under the sleeve for the escape of fluid when the pressure of fluid issuing from said outlet urges the sleeve away from that portion of the insert. The sleeve is preferably of elastomeric material. The transverse cross-section of the concave surface portion preferably increases in the direction away from said outlet. The insert is desirably of generally flat shape over at least the fluid escape portion extending from said outlet, the concavity being formed in one of the faces of the flat part of the insert. The fluid inlet to the insert is conveniently situated at the end thereof remote from the concave surface portion. The inlet end portion of the insert is suitably of circular cross-section. The sleeve may extend beyond the insert at the fluid escape end, so that the fluid escapes from the end of the channel into the region of the sleeve beyond the insert.

In another form the collapsible conduit comprises a flexible tube which will itself collapse to substantially zero internal cross-section. Preferably the tube comprises two flat layers of flexible material sealed together face-to-face along opposite longitudinal edges. Separate layers may be sealed together, or the layers may be opposite surfaces of a flattened tube which has been subjected to a sealing process along its opposite edges.

The device may further comprise an enclosure for the collapsible conduit, the enclosure having inlet means for the communication thereto of externally applied fluid pressure, whereby the internal cross-section of the conduit is controlled by the action of the externally applied pressure.

In the intravenous infusion set a constriction device may be connected with the first tube for setting the liquid flow rate through the collapsible conduit. Where a said enclosure is provided for the collapsible conduit, an inlet to the enclosure may be connected with a conduit for fluid pressure transfer connection with the liquid in the giving bag or bottle, whereby variations in the pressure head of the liquid in the giving bag or bottle are transmitted as corresponding variations in pressure applied to the collapsible conduit, tending to maintain a constant rate of flow of liquid through the collapsible conduit. A chamber may be provided in said fluid pressure transfer conduit, the chamber having a lower port for liquid leading from the giving bag or bottle and an upper port for gas leading to the enclosure, whereby gas pressure in the enclosure varies with the difference in the level of liquid in the giving bag or bottle and that in the chamber. The height of the chamber is preferably adjustable for setting the liquid flow rate through the collapsible conduit.

In order that the invention may be more clearly understood, various embodiments will now be described with reference to the accompanying drawings, wherein:

Fig. 1 is a diagrammatic side view of an intravenous infusion set embodying the invention,

Figs. 2 and 3 are side and edge views of a rigid insert for the collapsible conduit,

Figs. 4 and 5 are cross-sections on the lines A-A and B-B respectively of Fig. 3,

Fig. 6 is a cross-sectional edge view of the collapsible conduit arrangement,

Figs. 7 and 8 are cross-sectional views on the line C-C of Fig. 6 in the open and collapsed conditions respectively of the sleeve,

Fig. 9 is a side view of the constriction device,

Fig. 10 is a cross-sectional view on the line D-D of Fig. 9,

Fig. 11 shows a front view of a second embodiment of infusion apparatus,

Fig. 12 shows an edge view of the collapsible conduit,

Fig. 13 shows a side view of the roller clamp of Fig. 11,

Fig. 14 shows a perspective view of the insert for the roller clamp of Fig. 13,

Fig. 15 shows a front view of a third embodiment of infusion apparatus,

Fig. 16 shows a front view of a fourth embodiment of infusion apparatus,

Fig. 17 shows a cross-sectional view of the constriction and bypass device of Fig. 16, and

Fig. 18 shows a detail cross-sectional view of the constriction and bypass device with the tube open.

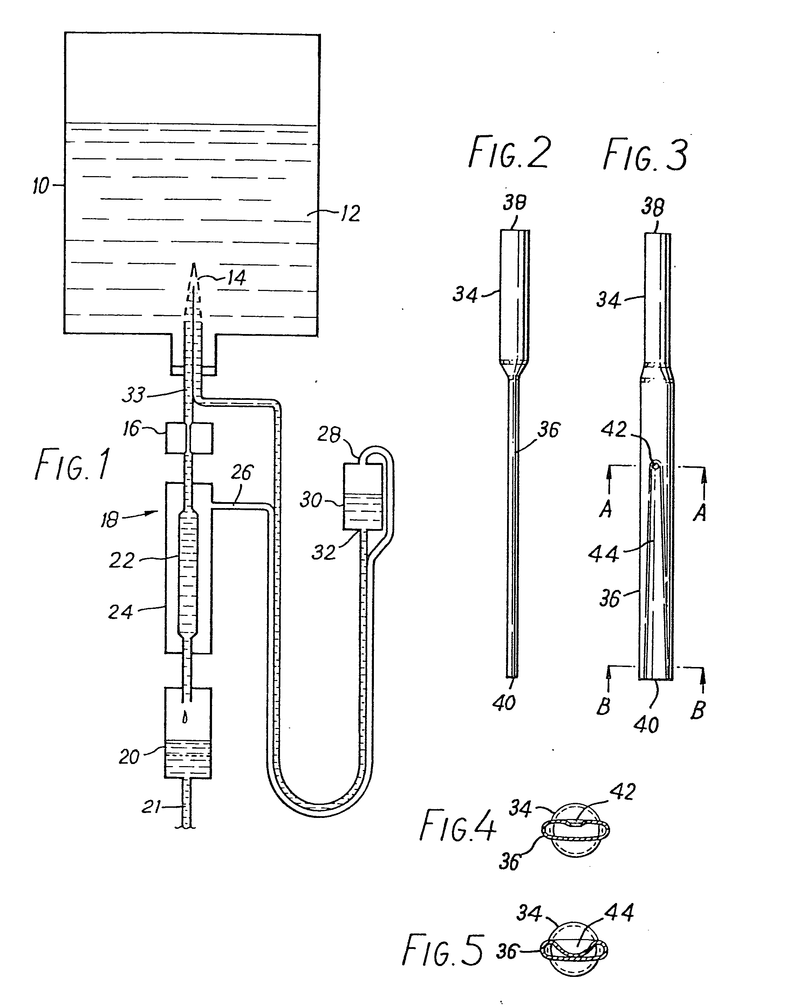

Referring firstly to Fig. 1; the apparatus comprises a giving bag or bottle 10 providing a reservoir for the liquid 12. A twin-lumen needle 14 provides a double outlet for the liquid from the reservoir. One outlet leads via a tube 33 to a constriction and bypass device 16, thence to a flow control device 18. From there the liquid passes to a conventional drip chamber 20 supplying the liquid via a tube 21 to the intravenous needle (not shown). The control device 18 comprises, essentially, a tube 22 which is collapsible for cutting off the flow of liquid therethrough, and an air-tight enclosure 24 having an inlet 26. This inlet 26 leads to an upper port 28 of a chamber 30, which is partly filled with liquid from the reservoir 10 entering the chamber through a lower port 32 supplied by the second outlet of the twin-lumen needle 14. The height of the chamber 30 is preferably adjustable relative to the reservoir 10.

The collapsible tube assumes a partially collapsed condition when there is zero pressure difference between the liquid attempting to enter the collapsible section and the gas pressure within the enclosure 24 (herein called the transmural pressure difference), or when there is excess pressure in the enclosure. Thus, the height of the chamber 30 is set so that the gas pressure in the enclosure 24 is equal to or greater than the liquid pressure attempting to enter the collapsible tube, and the tube is thereby in a partially collapsed condition. By this means, liquid flows into the collapsible tube at a rate determined by the transmural pressure difference set by the height of the chamber 30, and flows therefrom down to the drip chamber. As the liquid level falls in the reservoir, the pressure of liquid entering the collapsible tube must also fall, but the gas pressure in the enclosure 24 falls a corresponding amount owing to the lowering of the difference in liquid levels between the reservoir 10 and the chamber 30. In this way, the transmural pressure difference should remain substantially constant, and therefore the flow rate should also remain substantially constant through the control device 18. In practice, however, it may be found that substantial variations in the pressure head (for example, up to 2 cm. of water) may be needed to drive a given infusion rate through the control device. Thus, the infusion rate for a given pressure head may vary by a corresponding amount (e.g. up to 0.06 m 1/min.). For this reason the rigid constriction 16 may be incorporated upstream of the control device, providing a substantial pressure drop (e.g. 20 cm. water) for a typical infusion rate. Then the pressure downstream of this constriction is the pressure head needed to drive the desired infusion rate through the control device. Variations in this head by 2 cm. water will cause the pressure drop across the constriction to vary by only 10%, with a correspondingly small variation in the infusion rate. Since the collapsible tube operates in a partially collapsed condition, the flow of liquid through it is analogous to flow over a waterfall, in that downstream pressure changes cannot be transmitted upstream. Thus, the control device decouples from the flow-controlling system everything downstream of the distensible tube, including venous pressure changes. If the venous pressure (or more accurately, the pressure head required to force liquid into the patient's vein) rises above a critical level, however, the collapsible tube becomes fully distended and the flow rate becomes dependent on venous pressure. This critical pressure head is approximately the height of the liquid reservoir above the patient's vein; the exact critical height varies with the infusion rate from that of the reservoir liquid level at high rates, to about 30 cm. below this level at the lowest rates. Thus, the reservoir can be mounted at such a height that the critical pressure is above any venous pressure likely to be encountered in practice.

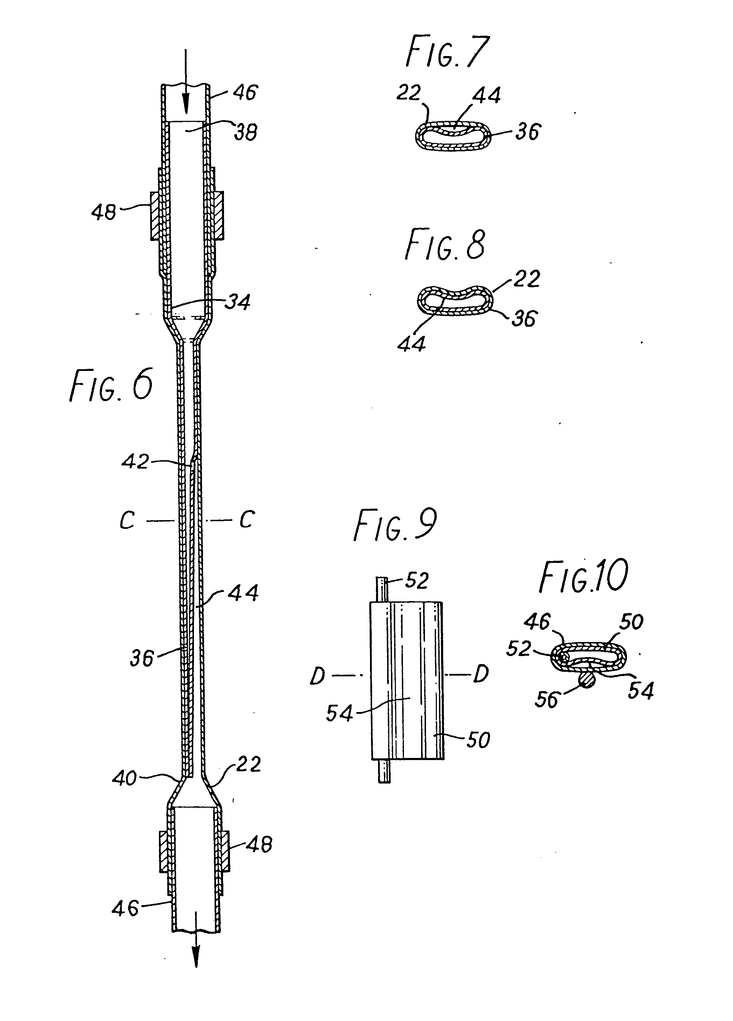

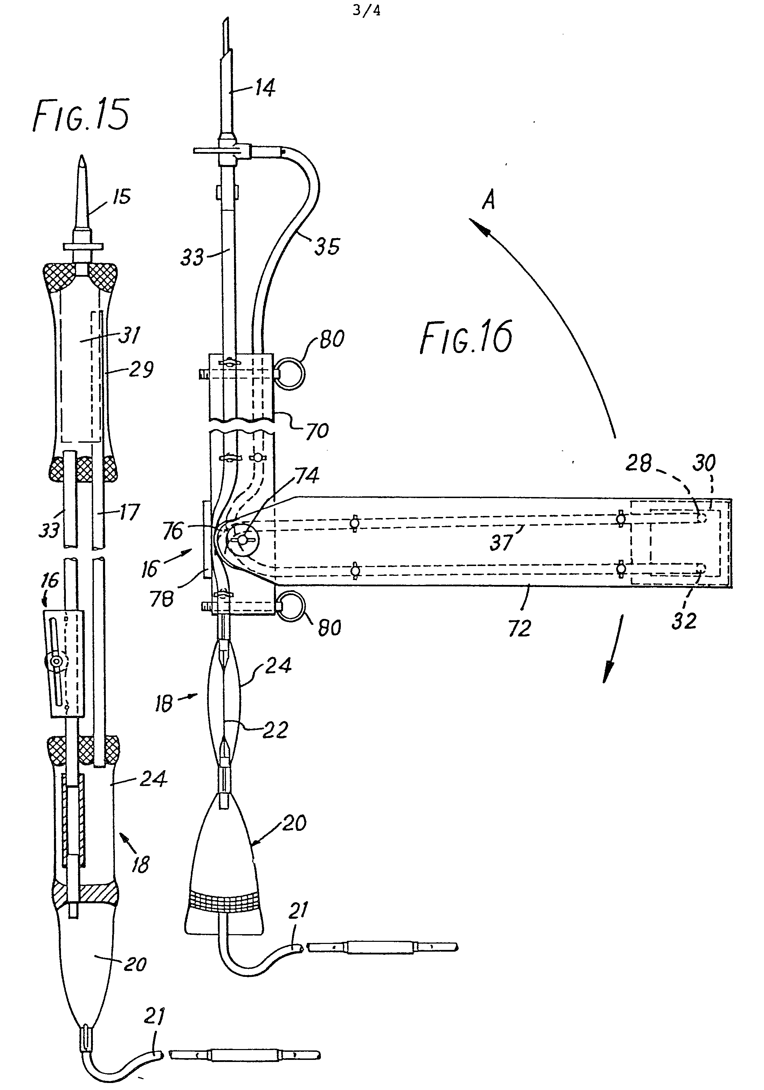

Referring to Figs. 2 to 8; the control device comprises an elastomeric latex tube 22; in this example it has an internal diameter of 0.65 cm. when uncollapsed at zero transmural pressure difference, and the walls are approximately 0.3 mm. thick. This tube surrounds a rigid insert 34, for example of copper tubing, originally 0.65 cm. internal diameter. The insert is shaped in the manner shown, by being substantially flattened over the greater part of its length 36. The insert is opened at the unshaped end 38 and closed at the flattened end 40. An aperture 42, 1.4 mm. in diameter, is formed in one face of the flattened portion, to provide an outlet for liquid entering the insert at 38. In addition, the same face of the insert is formed with a longitudinal concave channel 44 extending from the outlet 42 to the end 40, as indicated in Figs. 4 and 5, the depth of the channel increasing steadily towards the end 40. The latex tube 22 is a snug fit over the insert, since the internal circumference of the tube is the same as the external circumference of the insert. Thus, the tube fits into the cavity of the insert in the manner shown in Fig. 8, with excess external pressure, but when sufficient liquid pressure is applied at the aperture 42 the tube is urged thereby out of the channel, as indicated in Fig. 7, so that the liquid flows freely down the channel to the end 40. At each end, the tube 22 fits over conventional tubing 46 for the liquid, and is sealed thereto by collars 48. The particular dimensions and materials of the control device described are given by way of example only, and may be varied as desired in different embodiments.

The constriction 16 can be provided by introducing into the conventional polyvinyl chloride tubing 46 for the liquid the short insert shown in Figs. 9 and 10.

This can be made from copper tubing 50, sealed at its ends, but with a fine stainless steel tube 52 passing through it. The insert has a concave face 54 and its external cross-section is a little larger than the bore of the polyvinyl chloride tube, so that there is a substantial channel formed by the concavity 54. Externally of the polyvinyl chloride tube 46 is located a steel rod 56 which can be moved so as to force the tubing 46 into the concavity 54 and seal the bypass provided thereby, so that the liquid must now flow through the fine tube 52, which provides the rigid constriction. The rod is held loosely in position, for example by flexible plastics sheeting, and locking forceps are used to force the rod into the concavity. The bypass is used for initial filling, and is closed when the apparatus is in use. It will be seen that, since the constriction device operates by a positive opening or closing of the bypass, it is not subject to "creep" as is the case with conventional adjustable roller clamps.

In this embodiment of the apparatus, the flow rate is unaffected by changes in intravenous pressure, the phenomenon of "creep" associated with conventional roller or other clamps used to adjust the flow rate, or the fall of liquid level in the reservoir. The flow rate is set by adjusting the height of the chamber 30 relative to the reservoir, and a scale graduated in flow rate can be provided to facilitate this. However, simpler arrangements are possible within the present invention, which do not provide the same degree of control, but which may be preferred in some instances. For example, the chamber 30 could be at a fixed level, and the flow rate controlled by a clamp. However, the clamp should keep the "creep" to a minimum. Even simpler is an arrangement which dispenses with the enclosure 24 altogether (and hence also the chamber 30 and its associated parts). In this case, the collapsible tube is incorporated in an otherwise standard drip set, and a clamp is used to adjust the flow rate. This means that the flow rate is susceptible to changes in the level of liquid in the reservoir, but is still independent of the venous pressure downstream.

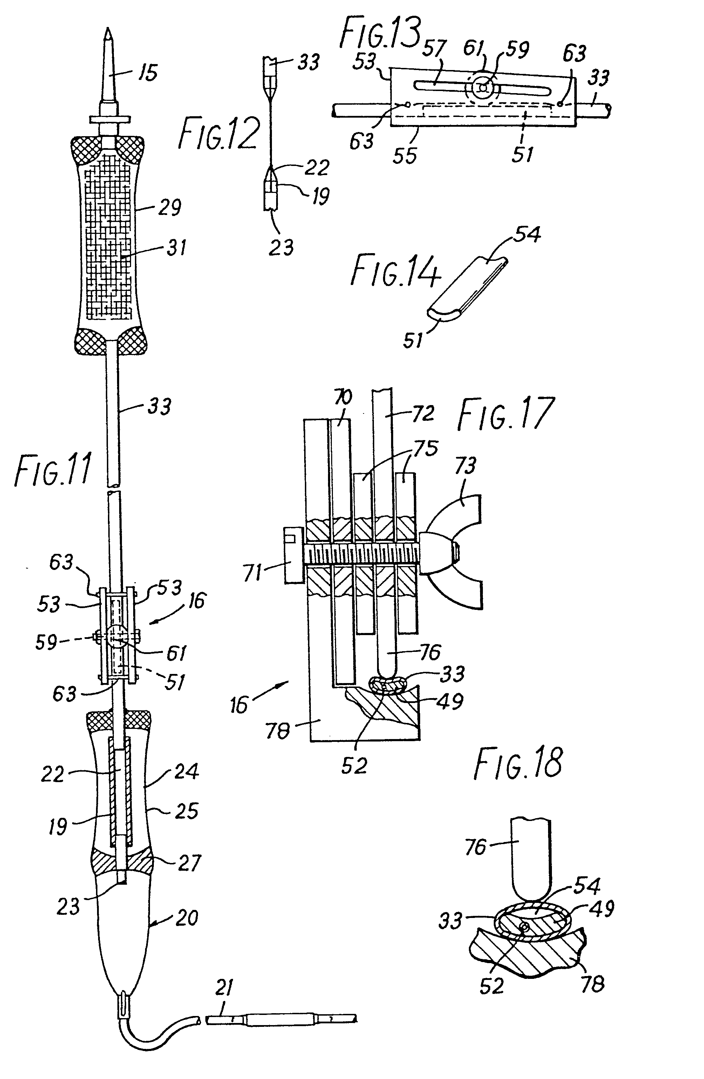

Referring to Fig. 11; this infusion apparatus has certain components in common with that shown in Fig. 1. A single needle 15 for insertion into a giving bag is connected to a chamber 29 made from polyvinyl chloride (p.v.c.) film and containing a filter 31. From the lower end of the chamber 29 p.v.c. tubing 33 extends via a constriction device 16 to a combined flow control device 18 and drip chamber 20, also made from p.v.c. film. From the drip chamber 20 extends p.v.c. tubing 21 which leads to a conventional intravenous needle (not shown). The flow control device 18 comprises an enclosure 24 with an opening 25 to atmosphere. The enclosure 24 is separated from the drip chamber 20 by a transverse weld 27 through which extends a short length of tubing 23. The tubing 33 extends a short distance into the top of the enclosure 24, and is connected to the tubing 23 by a collapsible conduit 22. This conduit 22 is made from two layers of flexible thin-walled p.v.c. film of which the opposed faces have been sealed along opposite sides at 19 leaving an unsealed central longitudinal strip. The two film layers can be separate layers brought together face-to-face or else formed from a length of p.v.c. tubing which has been flattened and the opposed faces sealed along opposite edges 19. Thus, the normal configuration for the tube 22 is flat, as indicated by the edge view as shown in Fig. 12. The ends of the tube are opened to fit over the tubes 33 and 23, and if necessary are sealed thereto. Thus, in the absence of any transmural pressure difference the central portion of the tube 22 remains flat, and thus closed against the transfer of liquid. The tube 22 will open as a result of the head of liquid in the tube 33 and chamber 29 from the giving bag, and will open to an extent determined by that liquid head, which thereby determines the rate of flow, subject to control by the constriction device 16.

As shown in Fig. 13, the constriction device 16 is of a roller clamp type. It comprises a channel section bracket having opposite walls 53 and a base 55, through which passes the tubing 33, the bracket being retained on the tube by pins 63 between the walls 53 which bear upon the tubing. The portion of the tubing 33 between the pins 63 contains an insert 51 (see Fig. 14) which is generally similar in external shape to that shown in Figs. 9 and 10, but does not have a constriction tube. Slots 57 are provided in the side walls 53 of the bracket, the slots slightly converging towards the base of the channel from one end of the bracket to the other. An axle 59 extends between the bracket walls 53 and its ends are located in the slots 57. In between the walls 53 the axle 59 carries a roller 61 of convex cross-section at its periphery, which bears upon the surface of the tubing 33 between the pins 63. Thus, by moving the axle along the slots 57 the roller 61 urges the tube 33 into the concave face 54 of the insert 51, so that towards one end of the slots 57 it closes the tube 33 completely. Because the roller 61 is pressing the tube 33 against an insert 51, the problem of creep associated with conventional roller clamps can be minimised. Thus, the clamp can be used to control the flow rate, and this embodiment represents a simple infusion set in which the effects of venous pressure changes and patient movement are decoupled from the flow rate by means of the flow control device 18.

The embodiment shown in Fig. 15 is in many respects similar to that shown in Fig. 11, and like parts are given the same reference numerals. In this embodiment, however, instead of the enclosure 24 being open to atmosphere through the aperture 25, it is connected by p.v.c. tubing 17 to the chamber 29. The upper end of the tubing 17 opens into the chamber 29 above the level of liquid therein, so that there is pressure transfer between the chamber 29 and enclosure 24. By this means, as discussed earlier in this Specification, the flow rate will also be independent of the variation in the liquid head as the level of liquid in the giving bag falls.

Referring to Figs. 16 to 18; this device is essentially a practical embodiment of the device shown diagrammatically in Fig. 1, and like parts are given the same reference numerals. From the twim lumen needle 14 extend two p.v.c. tubes 33,35 respectively, each mounted to a common support board 70. The tube 33 passes through a constriction and bypass device 16 to a flow control device 18 and drip chamber 20 via a collapsible tube 22 in an enclosure 24 of the flow control device 18. The flow control device is essentially similar to that shown in Fig. 15, except that it is seen edge on in Fig. 16. The tube 35 takes liquid from the giving bag to a chamber 30 which is made from welded p.v.c. film, and from the upper part of the chamber 30 a p.v.c. tube 37 extends to the enclosure 24 to provide pneumatic communication between the enclosure 24 and chamber 30. Because the embodiment of Fig. 16 is seen edge on, the entry of the tube 37 into the enclosure 24 is not visible, but it is essentially the same as shown in Fig. 15. The chamber 30 and the portions of tubing 35,37 connected thereto are mounted to a support arm 72, the chamber 30 being at one end of the arm and the other end of the arm being pivotally mounted at 74 to the board 70. The nose of the arm 72 around the pivot point 74 is formed as a cam 76 which co-operates with an abutment plate 78 on the board 70 so as to compress the tubing 33 between the cam 76 and plate 78 as the arm 72 is swung downwardly from its most raised position A. This is shown in more detail in Figs. 17 and 18. The arm 72 is pivotally mounted to the board 70 by means of a screw 71 and wing nut 73, with annular spacer members 75 sandwiching the nose 76 of the arms 72. With the wing nut 73 moderately tightened the arm 72 is held securely by friction between the cam 76 and tubing 33 in any desired angular position relative to the board 70. An insert 49, similar to the insert 51 shown in Fig. 14 but with a fine tube 52 passing through it (i.e. somewhat similar to the insert of Figs. 9 and 10), is located in the tubing 33 between the cam nose 76 and the abutment plate 78, the concave face of the insert facing the cam 76, and the plate 78 being somewhat concave to form a suitable seating. Fig. 17 shows the situation when the arm 72 is below the fully raised position A so that the cam 76 has pushed the tubing 33 firmly into the concave portion of the insert 49. In these positions, the liquid can only pass through the fine tube 52. Fig. 18, however, shows the position when the arm is fully raised, so that the liquid can bypass the constriction tube 52 and flow in the space between the insert 49 and the tube 33; this position being used for flushing through the system.

The device is used in the same way as that shown in Fig. 1. The board 70 is mounted to a support, for example a pole, by means of elastic bands 80. By raising or lowering the arm 72 through the various positions below the flushing position A the pressure in the enclosure 24 can be varied, which thereby varies the flow rate. Since the arm 72 is clamped in position by the wing nut 73, and the tubing 33 is fully pressed towards the insert 49, the constriction afforded by the device 16 and the set flow rate are not subject to creep.

Of course, although the invention and its various modifications have been particularly described in relation to an intravenous infusion set, it could be applied in other fields, for example mechanical blood pumps, artificial lung ventilation, and even industrial processes involving gas or liquid flow control. It should be appreciated that the collapsible tube is used with an insert or is specially constructed so that it can collapse to cut off fluid flow. An ordinary flexible tube will not collapse completely, but could be used on its own in applications where the flow rate is to be controlled only down to levels at which the tube cross-section still varies with the flow rate. In this condition the "waterfall" effect, described above, can still occur, thus preventing the propagation of pressure variations from below this region against the liquid flow.

CLAIMS:

1. A method of maintaining a desired rate of fluid flow, characterised in that the fluid (12) is passed along a path which includes a collapsible conduit (22) which can vary readily in internal cross-section according to variations in the transmural pressure difference, and arranging that the pressure head driving the fluid (12) through the fluid path downstream of the collapsible conduit is sufficiently smaller than the pressure head driving the fluid through the collapsible conduit, that the collapsible conduit is partially collapsed and mean fluid pressure variations in the downstream path are not propagated upstream of the collapsible conduit.

2. A method according to claim 1 wherein an enclosure (24) is provided surrounding the collapsible conduit, the internal cross-section of the conduit being controlled by the action of external fluid pressure applied to the enclosure.

3. A method according to claim 2 for maintaining a rate of liquid flow, wherein the collapsible conduit is connected to a reservoir (10) for the liquid, an inlet (26) to the enclosure (24) being connected with a conduit in fluid pressure transfer connection with the liquid in the reservoir (10), whereby variations in the pressure head of the liquid in the reservoir are transmitted as corresponding variations in pressure applied to the collapsible conduit, tending to maintain a constant rate of flow of liquid through the collapsible conduit.

4. A method according to claim 3 wherein a chamber (30) is provided in said fluid pressure transfer conduit between the liquid reservoir (10) and the inlet (26) to the enclosure, the chamber having a lower port (32) for liquid leading from the reservoir and an upper port (28) for gas leading to the enclosure, whereby gas pressure in the enclosure varies with the difference in the level of liquid in the reservoir (10) and that in the chamber (30), the height of the chamber (30) preferably being adjustable relative to the reservoir for setting the liquid flow rate through the collapsible conduit.

5.   A method according to any one of the preceding claims wherein the collapsible conduit comprises a flexible sleeve (22) surrounding a rigid insert (34), the internal circumference of the sleeve in cross-section being substantially the same as the external circumference of the cross-section of the insert which it surrounds, the insert having an inlet (38) leading to an internal passage for the fluid and a lateral outlet (42) therefrom intermediate the ends of the insert and covered by the sleeve, a longitudinal surface of a portion (36) of the insert from the region of said outlet being concave in cross-section to provide a channel (44) under the sleeve for the escape of fluid when the pressure of fluid issuing from said outlet (42) urges the sleeve away from that portion of the insert.

6.   A method according to claim 5 including any one or more of the following features, namely:
   (a) the sleeve (22) is of elastomeric material;
   (b) the transverse cross-section of the channel (44) in the portion (36) of the insert (34) increases in the direction away from said outlet (42);
   (c) the insert (34) is of generally flat shape over at least the fluid escape portion (36 ) extending from said outlet (42), the concavity (44) being formed in one of the faces of the flat part of the insert;

(d) the fluid inlet (38) to the insert is situated at the end thereof remote from the concave surface portion (44).

7. A method according to any one of claims 1 to 4 wherein the collapsible conduit (22) comprises a flexible tube (Figs. 11 and 12) which can itself collapse to substantially zero internal cross-section; the flexible tube preferably comprising two flat layers of flexible material sealed together face-to-face along opposite longitudinal edges (19), the two layers being separate layers of flexible material or opposite surfaces of a flattened tube which has been subjected to a sealing process along its opposite edges (19).

8. An intravenous infusion set adapted for maintaining a desired rate of liquid flow by a method according to any one of claims 1 to 7, the set comprising a first tube (33) for connection to a giving bag or bottle, a second tube (21) for connection to a hypodermic needle, and a drip chamber (20) and a said collapsible conduit (22) located in the liquid path between the two tubes (21,33).

9. Apparatus for maintaining a desired liquid flow rate by a method according to claim 3, the apparatus including a collapsible conduit (22), a tube connecting the collapsible conduit to a reservoir (10) of the liquid, an enclosure (24) surrounding the collapsible conduit, the enclosure having inlet means (26) for the communication thereto of externally applied fluid pressure, whereby the internal cross-section of the conduit (22) can be controlled by the action of externally applied fluid pressure, the inlet to the enclosure being connected with a conduit in fluid pressure transfer connection with said reservoir (10), whereby variations of the pressure head of the liquid in the reservoir are transmitted as corresponding variations in pressure applied both internally and externally to the collapsible conduit (10) tending to maintain a constant rate of flow of liquid through the collapsible conduit; optionally a chamber (30) being provided in said fluid pressure transfer conduit, the chamber having a lower port (32) for liquid leading from the reservoir (10) and an upper port (28) for gas leading to the enclosure (24), whereby gas pressure in the enclosure varies with the difference in the level of liquid in the reservoir (10) and that in the chamber (30).

10. Apparatus for maintaining a desired liquid flow rate by a method according to claim 5 or claim 6, including a collapsible conduit comprising a flexible sleeve (22) surrounding a rigid insert (34), the internal circumference of the sleeve in cross-section being substantially the same as the external circumference of the cross-section of the insert which it surrounds, the insert having an inlet (38) leading to an internal passage for the fluid and a lateral outlet (42) therefrom intermediate the ends of the insert and covered by the sleeve (22), a longitudinal surface of the portion (36) of the insert from the region of said outlet (42) being concave in cross-section to provide a channel (44) under the sleeve for the escape of fluid when the pressure of fluid issuing from said outlet (42) urges the sleeve away from that portion of the insert.

0034671

1/4

FIG.1

FIG.2   FIG.3

FIG.4

FIG.5

*FIG.7*

22 44 36

*FIG.8*

22 36 44

*FIG.6*

46 38 48 34 42 44 36 40 22 48 46

C — C

*FIG.9*

52 D — D 54 50

*FIG.10*

46 50 52 56 54

0034671

3/4

FIG.15

FIG.16

FIG.13

FIG.12

FIG.14

FIG.17

FIG.11

FIG.18

European Patent
Office

**EUROPEAN SEARCH REPORT**

EP 80 30 0459

| Category | DOCUMENTS CONSIDERED TO BE RELEVANT | | CLASSIFICATION OF THE APPLICATION (Int. Cl. 3) |
|---|---|---|---|
| | Citation of document with indication, where appropriate, of relevant passages | Relevant to claim | |
| | US - A - 4 186 740 (GUERRA) <br> * figures 1,3,5,11A, 11B; column 2, lines 6-14 and last two lines; column 3, complete; column 4, lines 1-30, 44-59; column 7, lines 41-59; column 8, lines 19-68; column 9, lines 1-12 * <br> --- | 1,2, 4,8 | A 61 M 5/14 <br> A 61 M 1/03 <br> G 05 D 7/01 <br> F 16 K 7/07 |
| | GB - A - 1 467 946 (SHIBAURA) <br> * figures 1-7; page 1, lines 10-14, 90-100; page 2, lines 1-26, 88-130; page 3, lines 1-6 * <br> --- | 1,2,7, 8 | |
| | FR - A - 1 022 192 (PROCEDES S.E.M.) <br> * figures 1-6; page 1, column 1, complete; column 2, lines 18-40; page 2, column 1, lines 1-40 * <br> --- | 1,2 | TECHNICAL FIELDS SEARCHED (Int.Cl. 3) <br><br> A 61 M <br> G 05 D <br> F 16 K |
| | US - A - 2 786 642 (COMB) <br> * figures 8-10; column 1, lines 15-20; column 6, lines 56-75; column 7, lines 1-43 * <br> --- | 2,5,10 | |
| | US - A - 2 766 765 (BOLANOWSKI) <br> * figures 1-5; column 1, lines 15-23; column 2, lines 14-31, 46-58; column 3, lines 20-72; column 4, lines 7-13 * <br> --- | 2,5 | |
| A | US - A - 4 043 332 (METCALF) <br> * column 2, lines 55-68 * <br> --------- | | |

CATEGORY OF CITED DOCUMENTS

X: particularly relevant

A: technological background

O: non-written disclosure

P: intermediate document

T: theory or principle underlying the invention

E: conflicting application

D: document cited in the application

L: citation for other reasons

&: member of the same patent family, corresponding document

The present search report has been drawn up for all claims

| Place of search | Date of completion of the search | Examiner |
|---|---|---|
| The Hague | October 20, 1980 | MAROSCIA |

EPO Form 1503.1  06.78